# EUROPEAN PATENT APPLICATION

(11) **EP 3 251 521 A1**
(43) Date of publication of application: **06.12.2017**
(21) Application number: 15879859.5
(22) Date of filing: 26.01.2015
(51) Int. Cl.: A23J 1/00

(54) **METHOD FOR PRODUCING HOP EXTRACT**

(71) Applicant: Suntory Holdings Limited, Osaka-shi, Osaka 530-8203 (JP)
(72) Inventor: ARITA, Tetsuya, Osaka 618-8503 (JP); HIDAKA, Koichiro, Kanagawa 211-0067 (JP); SHIBUYA, Katsushi, Osaka 618-0001 (JP)
(74) Representative: Vossius & Partner Patentanwälte Rechtsanwälte mbB
(86) International application number: PCT/JP2015/051988
(87) International publication number: WO 2016/120962

(57) **Abstract**

A method for producing a hop extract including filtering a hop-containing water at a temperature of 50°C or higher, to give a hop extract. According to the method of the present invention, a hop extract having a high polyphenol concentration can be conveniently prepared, and is suitably used as raw materials for compositions for treating and/or ameliorating proteotoxin neutralization, inhibition of enamel decalcification, body fat modulation, protection of periodontal membranes, virus inactivation, inhibition of overexpression of thymic stromal lymphopoietin, symbiotic regeneration, obesity, anti-inflammation, inflammatory bowel disease, coronary artery disease, hyperuricemia, allergic diseases, digestive diseases, and the like.

## Description

### TECHNICAL FIELD

The present invention relates to a method for producing a hop extract. More specifically, the present invention relates to a method for producing a hop extract suitably usable as raw materials for foodstuff, a hop extract obtainable by the production method, and foodstuff using the hop extract.

### BACKGROUND ART

Hops, for example, contain not only ingredients that give bitterness to beer-taste beverages but also ingredients that give refreshing hoppy aroma and a body. Specifically, bitterness is attributable to an α-acid or the like in the hops, hoppy aroma attributable to terpenes or the like, and a body attributable to polyphenols or the like, so that each of them is attributable to various ingredients. Accordingly, it has been made possible to adjust the quality of the foodstuff using hops as raw materials by selecting the hops and processing them.

For example, Patent Publication 1 discloses a pre-isomerization method of hops including continuously passing pre-dried pressed hops through a steamer-extruder having at least two screws together with water in an amount of 50% by mass or less, to carry out steaming and extrusion of the hops at a temperature lower than 120°C. By taking the isomerization method described above, yield of isomerization is improved, so that high-quality beer manufactured articles can be obtained.

In addition, Patent Publication 2 discloses a method including metabolizing an aqueous residue in wastewater generated in food processing factories with bacteria to convert the aqueous residue to an appropriate component for use in animal feeds. As the residue, hops are exemplified.

### RELATED ART REFERENCES

### PATENT PUBLICATIONS

Patent Publication 1: Japanese Patent Laid-Open No. Sho-63-196260
Patent Publication 2: Japanese Unexamined Patent Publication No. 2011-502485

### SUMMARY OF THE INVENTION

### PROBLEMS TO BE SOLVED BY THE INVENTION

Generally, the hop processed products include "Dry Hop Flowers" in which hops are simply dried; "Hop Pellets Type 90" in which Dry Hop Flowers are pulverized and pelletized; "Hop Pellets Type 45" in which Dry Hop Flowers are frozen and pulverized, and a fractionated lupulin fraction is concentrated to form into pellets; and "Hop Extract" in which Hop Pellets Type 90 are subjected to a CO₂ extraction to extract bitterness ingredients. Further, as processed products having high degree of purification, as the aroma giving, "Oil-Rich Extract" in which hop pellets are subjected to an ethanol extraction to extract aroma ingredients can be used; as the body giving, "Polyphenol-Rich Pellets" which are by-products discharged during the production of hop extracts, and "Polyphenol-Rich Extract" obtained by concentrating only the polyphenol fraction from Polyphenol-Rich Pellets, and the like can be used.

However, the hop processed products obtained by conventional processing methods do not yet sufficiently satisfy the content of the desired ingredient. Also, although the processed products of extract types such as "Oil-Rich Extract" and "Polyphenol-Rich Extract" are processed products having high degree of purification, the costs are enormous, making it disadvantageous in applications to foodstuff.

An object of the present invention is to provide a production method for conveniently obtaining a hop extract having a high polyphenol content, a hop extract obtainable by the production method, and foodstuff using the hop extract.

### MEANS TO SOLVE THE PROBLEMS

In view of the above, as a result of intensive studies in order to solve the above problems, the present inventors have found that when the hop extract is prepared by carrying out extraction procedures of hops, and then carrying out a removal treatment of the residue, it is made possible to increase the polyphenol content of the hop extract obtained by filtering a treatment liquid to be subjected to a removal treatment of the residue at a temperature of 50°C or higher, and the present invention has been perfected thereby.

The present invention relates to the following [1] to [3]:
[1] A method for producing a hop extract, including filtering a hop-containing water at a temperature of 50°C or higher, to give a hop extract.
[2] A hop extract produced by a method as defined in the above [1].
[3] Foodstuff containing a hop extract obtained by a method as defined in the above [1].

### EFFECTS OF THE INVENTION

The hop extract obtainable by the method of the present invention exhibits an excellent effect of having a high polyphenol content.

### BRIEF DESCRIPTION OF THE DRAWINGS

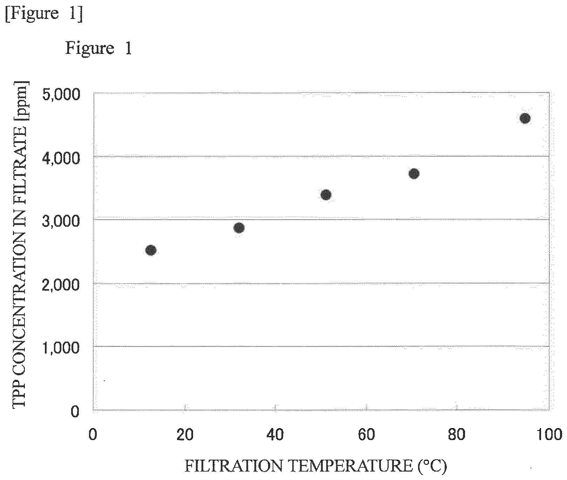

[FIG. 1] FIG. 1 is a graph showing the relationship between a temperature of a hop-containing water during filtration and T-PP concentration in the filtrate.

### MODES FOR CARRYING OUT THE INVENTION

The method for producing a hop extract of the present invention has a feature of including the step of filtering a hop-containing water at a temperature of 50°C or higher. In other words, in the present invention, the present inventors have found for the first time that when an extract is obtained by removing a residue from a mixture of hops and water, a liquid mixture containing the residue of which temperature is 50°C or higher is filtered, whereby a polyphenol content of the extract obtained increases. Although the detailed reasons are not elucidated, the influences of solubilities of the eluting components containing polyphenol in the hops are considered, and it is considered that a polyphenol is dissolved in the hop-containing water at a temperature of 50°C or higher, and would not be removed by filtration as a residue. In addition, the influences by the degree of adhesion of the polyphenol to the residue are also considered, and it is considered that a polyphenol is eluted in the hop-containing water at a temperature of 50°C or higher, and less likely to be adhered to the residue, so that the polyphenol is less likely to be removed together with the residue. Here, even if our assumptions were slipped, it would not affect the protected scope of the present invention. Conventionally, in order to increase an yield of a polyphenol, contrivances have been made in the extraction conditions in a manner that a polyphenol is extracted in a larger amount in the hop-containing water, but the filtration after the extraction has not been studied in depth. In the present invention, it has been succeeded in increasing the yield of a polyphenol in the hop-containing water by a very convenient method of temperature-controlling during the filtration. In general, the efficiency of the filtration is adjusted by the specifications of a filter material, a pressure applied to the filter material, and the specifications of a filtration aid (an agent to be used together with the filter material in order to efficiently perform filtration), but the findings of remarking on the temperature of a liquid to be filtered (liquid mixture containing a residue) are surprising, which were not known before the present inventors have arrived at the findings. Here, in the present specification, the polyphenol means a total polyphenol (T-PP), and can be measured in accordance with a method described in "The Methods of Analysis of BCOJ (November 1, 2004, revised edition), 7.11 Total Polyphenol" as prescribed by Brewery Association of Japan, Brewery Convention of Japan (BCOJ).

The method of the present invention includes the step of filtering a hop-containing water at a temperature of 50°C or higher.

The hop-containing water in the present invention refers to a mixture containing a hop and water as raw materials. In other words, the hop-containing water in the present invention refers to not only a mixture in which a hop and water are simply mixed to elute ingredients in the hop, but also a mixture in which a hop and water are mixed and then subjected to some sort of treatment, among which a mixture in which a hop and water are mixed, then heated, and subjected to extraction procedures is preferred.

As the hop usable in the present invention, a hop flower thereof (matured non-pollinated pistillate flower) in its entirety is directly used or crushed, or a bract part richly containing a polyphenol component can be selected and used. In addition, hop processed products such as "Dry Hop Flowers" in which hop flowers are simply dried; "Hop Pellets Type 90" in which Dry Hop Flowers are pulverized and pelletized; dried products of hop bracts, which are flower petal-like tissues outside of the hop flower; pelletized products of crushed hop bracts, and the like can also be used. Here, the kinds of the hops may be identical or different, and two or more kinds may be used in combination. The shape thereof is also not particularly limited.

In addition, in the present invention, other solvents besides water can be used within the range that would not impair the effects of the present invention. Other solvents may be aqueous solvents, and a water-containing alcohol or the like can be used. The content of water in all the solvents including water, when considering the blending to foodstuff, is preferably 99.5% by mass or more, more preferably 99.8% by mass or more, and even more preferably 99.9% by mass or more. Here, all the solvents including water and other solvents as used herein may be collectively simply referred to as an extraction solvent.

The pH of the extraction solvent is, but not particularly limited to, preferably from 4.5 to 9.5, and more preferably from 5.0 to 8.5.

A mass ratio of water to a hop (hop solid content) subjected to extraction (water/hop solid content) is preferably from 500/66.6 to 500/22.2, more preferably from 500/60 to 500/30, and even more preferably from 500/55 to 500/35, from the viewpoint of efficiently collecting a high-concentration liquid. Here, the hop solid content as used herein means a weight of a hop before addition on a dry basis.

The treatment temperature with an extraction solvent is not particularly limited, so long as the treatment temperature is a temperature capable of extracting a polyphenol from the hop. The treatment temperature is preferably 60°C or higher, more preferably 70°C or higher, and even more preferably 80°C or higher, from the viewpoint of high yield. In addition, the treatment temperature is preferably 100°C or lower, more preferably 99.5°C or lower, and even more preferably 99°C or lower.

Although the treatment time with the extraction solvent depends upon the treatment temperatures, for example, in a case where the treatment temperature is from 95° to 100°C, the treatment time is preferably from 10 to 90 minutes, and more preferably from 60 to 90 minutes.

The hop-containing water obtained is subjected to a filtration treatment in order to remove extraction residues, and if the temperature of a treatment liquid to be subjected to a filtration treatment would be 50°C or higher, the hop-containing water immediately after the extraction treatment may be subjected to a filtration treatment, or alternatively a cooled hop-containing water may be again heated to a liquid temperature of 50°C or higher, and then subjected to the filtration treatment.

As the method for filtration, a known method can be used without particular limitations, and pressure filtration in which a hop-containing water is subjected to filter filtration while pressurizing is preferred, from the viewpoint of collecting the hop-containing water from the residues in a high yield. In the present invention, for example, a pressure filtration method using a filtration apparatus such as a filter press or a screw press is preferred, from the viewpoint of carrying out even pressurization while securing the level of pressure.

The filter used is not particularly limited so long as the filter can filter a liquid of which liquid temperature is 50°C or higher, and the mesh size thereof is preferably 30 µm or more, and more preferably 50 µm or more, from the viewpoint of surely removing the residues, which are solid contents. In addition, the mesh size is 400 µm or less, more preferably 350 µm or less, and even more preferably 300 µm or less.

In addition, the flow rate of the treatment liquid that passes through a filter depends upon the kinds of the filtration apparatus, filter areas, amounts of treatment liquid, and the like, and can be properly adjusted in accordance with a known technique. In a case where a pressure filtration device is used, the applied pressure can be appropriately set.

The temperature of the hop-containing water to be subjected to filtration is 50°C or higher, and the temperature is preferably 60°C or higher, more preferably 70°C or higher, and even more preferably 80°C or higher, from the viewpoint of collecting a polyphenol in high yields. In addition, the temperature is preferably 100°C or lower, more preferably 99°C or lower, and even more preferably 98°C or lower. The preferred aspect of the present invention is in that the content of the polyphenol in the filtrate obtained can be controlled by controlling the temperature of the hop-containing water. Generally, the yields in filtration have been adjusted by the specifications of the filter material, applied pressure to the filtration agent, and the specifications of the filtration aid (an agent to be used together with the filtration agent in order to efficiently perform filtration). However, in the present invention, the content of the polyphenol in the filtrate obtained can be controlled by simply controlling the temperature of the hop-containing water while satisfying the basic functions of filtration of residue removal, so that there are no needs to setup again the conditions for filtration treatment such as specifications of filtration agent. For this reason, after the termination of filtration, the conditions of filtration facilities do not have to be setup again upon performing the subsequent filtration procedures. In addition, consequently, the polyphenol can be collected in the hop extract stably and in high yields. Here, the temperature of the hop-containing water in a case where a filtration apparatus such as a filter press or a screw press is used means a set temperature of the filtration device.

The filtrate after the filtration treatment may be subjected to a treatment such as centrifugation, filtration, ultrafiltration, or concentration, in accordance with a known method, and the filtrate after the treatment is also one embodiment of a hop extract of the present invention.

Thus, a hop extract is obtained. The hop extract obtained from which an extraction residue is removed through the filtration treatment is treated in an elevated temperature state while being clear, so that its polyphenol content is high. The polyphenol concentration in the hop extract is preferably 1,000 ppm or more, more preferably 1,200 ppm or more, and even more preferably 1,500 ppm or more. In addition, the polyphenol concentration is preferably 5,000 ppm or less, more preferably 4,700 ppm or less, and even more preferably 4,600 ppm or less.

In addition, the bitterness units of the hop extract are preferably 40 BUs or more, more preferably 60 BUs or more, and even more preferably 80 BUs or more, from the viewpoint of further processing the hop extract to be used as a bitterness agent. In addition, the bitterness units are preferably 600 BUs or less, more preferably 575 BUs or less, and even more preferably 550 BUs or less. Here, the bitterness units as used herein mean bitterness units measured in accordance with a method described in the section 8.15 Bitterness Units of The Methods of Analysis of BCOJ.

Here, the filtration residue of the hop-containing water after obtainment of the hop extract has a water content of preferably 85% by weight or less, more preferably 83% by weight or less, and even more preferably 80% by weight or less, from the viewpoint of collecting a hop extract from a residue in high yields, and drying the filtration residue at a low cost. The lower limit is not particularly limited, and is, for example, 50% by weight or more. Here, the water content of the filtration residue can be calculated by evaporating water in the residue, and measuring solid weights before and after evaporation.

The present invention also provides a hop extract obtained by the method of the present invention. Since the hop extract has a high polyphenol content, the hop extract can be suitably used as raw materials for, for example, pharmaceutical compositions, foodstuff, cosmetics, or feeds.

The pharmaceutical composition includes, for example, therapeutic agents or prophylactic agents of diseases in mammals such as human. Specifically, the hop extract of the present invention can be suitably used as raw materials for proteotoxin neutralizer, materials for inhibiting enamel decalcification, body fat modulators, protectants of periodontal membranes, virus-inactivating agents, inhibitors of overexpression of thymic stromal lymphopoietin, symbiotic regenerative agents, or antiinflammatories, or raw materials for therapeutic agents or prophylactic agents for inflammatory bowel diseases, coronary artery diseases, obesity, hyperuricemia, allergic diseases, and digestive diseases. The content of the hop extract of the present invention in the pharmaceutical composition is not particularly limited, and, for example, the hop extract of the present invention can be blended so that the polyphenol content is preferably from 1 to 1,000 ppm, more preferably from 5 to 500 ppm, and even more preferably from 10 to 300 ppm.

The foodstuff include, for example, foodstuff for ameliorating or preventing a state caused by the lowering of a polyphenol content in a mammal such as human. Specifically, the hop extract of the present invention can be suitably used as raw materials for foodstuff for ameliorating or preventing symptoms such as inflammatory bowel diseases, coronary artery diseases, obesity, hyperuricemia, allergic diseases, or digestive diseases. The content of the hop extract of the present invention in the foodstuff is not particularly limited, and the hop extract of the present invention can be blended so that, for example, the polyphenol content is preferably from 1 to 1,000 ppm, more preferably from 5 to 500 ppm, and even more preferably from 10 to 300 ppm.

Specifically, the foodstuff include those in the forms of tablets, granules, liquids, capsules, pills, powders, candies, drops, troches, gums, powder juice, drink agents, seasonings, processed foods, and the like. These can be suitably used as supplements. In addition, since the polyphenol content is increased, some excellent effects of being capable of adjusting flavors, such as being capable of giving body to the foodstuff obtained are exhibited.

The cosmetics include, for example, cosmetics such as whitening cosmetics and cosmetic oral agents for preventing and/or treating skin disorders. The content of the hop extract of the present invention in the cosmetics is not particularly limited, and, for example, the hop extract can be blended so that the polyphenol content is preferably from 1 to 1,000 ppm, more preferably from 5 to 500 ppm, and even more preferably from 10 to 300 ppm.

The feeds include, for example, feeds for improving or preventing a state caused by lowering the polyphenol content in livestock such as cows and bulls, pigs, fowls, sheep, and horses, and pet animals such as dogs and cats. Specifically, the hop extract of the present invention can be suitably used as raw materials for feeds for ameliorating or preventing various diseases such as hypercholesterolemia, hypertriglyceridemia, impaired glucose tolerance, hypertension, and obesity. The content of the hop extract of the present invention in the feeds is not particularly limited, and, for example, the hop extract can be blended so that the polyphenol content is preferably from 1 to 1,000 ppm, more preferably from 5 to 500 ppm, and even more preferably from 10 to 300 ppm.

The pharmaceutical composition, the foodstuff, the cosmetics, and the feed mentioned above can be prepared by appropriately blending carriers, base agents, and/or additives or the like ordinarily used in pharmaceutical fields or food fields, and the like, within the range that would accomplish the objective of the present invention, so long as the above hop extract of the present invention is blended therewith.

### EXAMPLES

The present invention will be specifically described hereinbelow by the Examples, without intending to limit the scope of the present invention to the following Examples.

### Examples (Levels A, B, and C) and Comparative Examples (Levels D and E)

Seventy grams of pelletized hops (produced in Czech Republic) were added to 700 g of a hot water heated to 98°C, and the mixture was then heated for 80 minutes, to give a hop-containing water. The water temperature at the termination of heating was 99.8°C.

The hop-containing water obtained was treated as follows. Level A was directly filtered with a mesh screen (screen sieve opening: 250 µm, 60 mesh). Each of Levels B to E was cooled to a temperature given below, and treated in the same manner as above. Thereafter, the hop draff containing the extract remaining on the mesh sieve was pressed onto the mesh sieve while pressurizing the bottom of the spatula with a hand, to collect a mesh-pass liquid. Each of the liquid temperatures upon filtration with the mesh screen was as follows. Level A (94.8°C), Level B (70.5°C), Level C (51.2°C), Level D (32.1°C), and Level E (12.7°C).

Here, the liquid temperature was measured with a digital thermometer SK-250WP II-N manufactured by SATO KEIRYOKI MFG. CO. LTD.

After the filtration with the mesh screen, the filtrate was centrifuged to give each of samples of Levels A to E.

For each of samples of Levels A to E, a polyphenol content was measured. Although the polyphenol content can be measured by any one of generally known methods, the polyphenol content was measured in accordance with "The Methods of Analysis of BCOJ (November 1, 2004, revised edition) 7.11 Total Polyphenol" as prescribed by Brewery Association of Japan, Brewery Convention of Japan (BCOJ). The results are shown in Table 1.
[Table 1]

**Table 1**

| | Level A | Level B | Level C | Level D | Level E |
|---|---|---|---|---|---|
| Amount of Hops Used (g) | 70 | | | | |
| Amount of Water Used (g) | 700 | | | | |
| Water/Hops (Mass Ratio) | 10 | | | | |
| Extraction Temperature (°C) | 98.0 - 99.8 | | | | |
| Extraction Time (minute) | 80 | | | | |
| Temperature During Pressure Filtration (°C) | 94.8 | 70.5 | 51.2 | 32.1 | 12.7 |
| T-PP of Extract (ppm) | 4,580 | 3,708 | 3,383 | 2,863 | 2,518 |

From Table 1, it could be confirmed that the contents are Level A (4,580 ppm), Level B (3,708 ppm), Level C (3,383 ppm), Level D (2,863 ppm), and Level E (2,518 ppm), so that Levels A, B, and C where a temperature during the filtration is 50°C or higher had a high polyphenol content.

### INDUSTRIAL APPLICABILITY

The hop extract obtainable by the method of the present invention has a high polyphenol concentration, and is suitably used, for example, as raw materials for compositions for treating and/or ameliorating proteotoxin neutralization, inhibition of enamel decalcification, body fat modulation, protection of periodontal membranes, virus inactivation, inhibition of overexpression of thymic stromal lymphopoietin, symbiotic regeneration, anti-inflammation, and the like.

## Claims

1. A method for producing a hop extract comprising filtering a hop-containing water at a temperature of 50°C or higher, to give a hop extract.

2. The method according to claim 1, wherein the filtration is a pressure filtration, and the hop extract comprises a polyphenol in an amount of from 1,000 to 5,000 ppm.

3. The method according to claim 1 or 2, wherein the hop-containing water which is heated to a temperature of from 95° to 100°C is subjected to an extraction treatment.

4. The method according to claim 3, wherein the heating time is from 10 to 90 minutes.

5. The method according to any one of claims 1 to 4, wherein a mass ratio of water to a hop solid content in the hop-containing water (water/hop solid content) is from 500/66.6 to 500/22.2.

6. The method according to any one of claims 2 to 5, wherein the pressure filtration is carried out with a filter press or a screw press.

7. The method according to any one of claims 1 to 6, wherein bitterness units of the hop extract measured in accordance with a method described in the section 8.15 Bitterness Units of The Methods of Analysis of BCOJ are from 40 to 600 BUs.

8. The method according to claim 6 or 7, wherein a mesh size of a filter used is from 30 to 400 µm.

9. The method according to any one of claims 1 to 8, wherein the temperature of the hop-containing water is from 60° to 100°C.

10. The method according to any one of claims 2 to 9, wherein the hop-containing water is heated to a temperature of 50°C or higher, and thereafter subjected to a pressure filtration.

11. The method according to any one of claims 1 to 10, wherein the water content of a filtration residue of the hop-containing water is 85% by weight or less.

12. A hop extract produced by a method as defined in any one of claims 1 to 11.

13. Foodstuff comprising a hop extract obtained by a method as defined in any one of claims 2 to 11.

14. The foodstuff according to claim 13, which are a supplement.
